Europäisches Patentamt

European Patent Office

Office européen des brevets

(19)

(11) **EP 0 637 932 B1**

(12) **EUROPÄISCHE PATENTSCHRIFT**

(45) Veröffentlichungstag und Bekanntmachung des Hinweises auf die Patenterteilung:
**07.01.1998 Patentblatt 1998/02**

(21) Anmeldenummer: **93909871.1**

(22) Anmeldetag: **30.04.1993**

(51) Int. Cl.$^6$: **A61B 5/028**

(86) Internationale Anmeldenummer:
**PCT/EP93/01052**

(87) Internationale Veröffentlichungsnummer:
**WO 93/21823 (11.11.1993 Gazette 1993/27)**

(54) **VORRICHTUNG ZUM BESTIMMEN DES FÜLLUNGSZUSTANDES EINES BLUTKREISLAUFS**

DEVICE FOR DETERMINING THE FILL LEVEL OF A BLOOD CIRCULATORY SYSTEM

DISPOSITIF POUR DETERMINER LE NIVEAU DE REMPLISSAGE DU SYSTEME CIRCULATOIRE SANGUINE

(84) Benannte Vertragsstaaten:
**DE ES FR GB IT**

(30) Priorität: **30.04.1992 DE 4214402**

(43) Veröffentlichungstag der Anmeldung:
**15.02.1995 Patentblatt 1995/07**

(73) Patentinhaber:
**PULSION Verwaltungs GmbH & Co
Medizintechnik KG
81675 München (DE)**

(72) Erfinder:
• **Pfeiffer, Ulrich, Dr.
D-81667 München (DE)**
• **Knoll, Reinhold
81675 München (DE)**

(74) Vertreter:
**WILHELMS, KILIAN & PARTNER
Patentanwälte
Eduard-Schmid-Strasse 2
81541 München (DE)**

(56) Entgegenhaltungen:
**EP-A- 0 060 546          WO-A-81/02512
WO-A-93/05704**

• **Database INSPEC, I.E.E., LONDON, G.B. INSPEC
No. 4031387 Computers in Cardiology, 19-22
Sept. 1989. C. Vasanelli et al. "Quantitative
angiographic identification of functional
ventricular aneurysms" siehe
Zusammenfassung**
• **F.R.LEWIS & U.J.PFEIFFER (EDS.) 'Practical
Applications of Fiberoptics in Critical Care
Monitoring' 1990 , SPRINGER-VERLAG , BERLIN
(DE) in der Anmeldung erwähnt siehe Seite 114 -
Seite 125**

## Beschreibung

Die Erfindung betrifft eine Vorrichtung zum Bestimmen des Füllungszustandes eines Blutkreislaufs insbesondere des globalen enddiastolischen Herzzeitvolumens GEDV, des intrathorakalen Blutvolumens ITBV, des pulmonalen Blutvolumens PBV, des extravasalen Lungenwasservolumens EVLW und/oder des globalen Herzfunktionsindex CFI, bei dem mittels Thermodilution das intrathorakale Thermovolumen ITTV und das pulmonale Thermovolumen PTV ermittelt werden.

Bei der intensivmedizinischen Diagnostik und Behandlung von schwerstkranken Patienten sind das Herzzeitvolumen C.O. und das Kreislauffüllungsvolumen wichtige Kenngrößen.

Weit verbreitet ist der Einsatz eines Pulmonalis-Einschwemmkatheters, mit dessen Hilfe sich das Herzzeitvolumen C.O. nach dem Thermodilutionsverfahren sowie, als Parameter für die Kreislauffüllung, der zentralvenöse Druck CVP, das enddiastolische Volumen der rechten Herzkammer RVEDV und der pulmonalkopilläre Verschlußdruck PCWP messen lassen. Hierzu siehe D. Payen "Physiological Determinants of Hemodynamic Parameters" aus "Strategy in Bedside Hemodynamic Monitoring", herausgegeben von J.-F. Dhainaut und D. Payen, Springer-Verlag, 1991, Seite 28 bis 35.

Die Messung der intravasalen Drucke CVP und PCWP gegenüber dem Athmosphärendruck hat den entscheidenden Nachteil, daß aus diesen Werten nicht sicher auf das zirkulierende Volumen geschlossen werden kann. Zudem reagieren die oft als Kreislauffüllungsdrucke bezeichneten Werte CVP und PCWP nicht sehr empfindlich auf Volumenänderungen. Hierzu siehe U.J. Pfeiffer et al. "Sensitivity of Central Venous Pressure, ..." aus F.R. Lewis und U.J. Pfeiffer "Practical Applications of Fiberoptics in Critical Care Monitoring", Springer-Verlag, 1990, Seiten 25 bis 31.

Aus dieser Erkenntnis heraus wurde die Messung des RVEDV-Wertes eingeführt, jedoch gibt dieser Wert nur Aufschluß über das Füllungsvolumen in einer der vier Herzkammern. Zudem weist die Bestimmung des RVEDV-Wertes eine erhebliche Schwankungsbreite auf. Hierzu siehe J.-E. Dhainaut et al. "Validity and Clinical Applications of Fast Response Thermistor for Right Ventricular Ejection Fraction Monitoring" aus J.-E. Dhainaut und D. Payen "Strategy in Bedside Hemodynamic Monitoring", Springer-Verlag, 1991, Seiten 59 ff..

Ein spezifischeres Verfahren zur Messung des Herzzeitvolumens C.O. und des Kreislauffüllungsvolumens ist die sogenannte Thermo-Dye-Technik. Diese Technik ist eine Kombination aus Farbstoffdilution DD und Thermodilution TD und stellt als spezifischen Parameter der Kreislauffüllung das intrathorakale Blutvolumen ITBV zur Verfügung. Dieser Wert umfaßt die enddiastolischen Volumina der rechten Vorkammer RAEDV und der rechten Hauptkammer RVEDV, das Blutvolumen in der Lunge (pulmonales Blutvolumen) PBV und die enddiastolischen Volumina der linken Vorkammer LAEDV und der linken Hauptkammer LVEDV. Dieses Verfahren hat weiterhin den Vorteil, daß auch der extravasale Wassergehalt der Lunge EVLW bestimmt werden kann. Hierzu siehe U.J. Pfeiffer et al. "A Fiberoptics-Based System ..." aus U.J. Pfeiffer "Practical Applications of Fiberoptics in Critical Care Monitoring", Springer-Verlag, 1990, Seiten 115 ff..

Das Thermo-Dye-Verfahren hat jedoch den Nachteil, daß es die Injektion eines relativ teuren Farbstoffes erfordert, wobei die injektionsfähige Farbstofflösung wegen der Licht- und Wärmeempfindlichkeit des Farbstoffes immer erst für die Injektion, d.h. mindestens einmal täglich, zubereitet werden muß. Zusätzlich kann der Farbstoff allergische oder anaphylaktische Reaktionen hervorrufen.

Der bei diesem Verfahren benutzte Faseroptik-Thermistor-Katheter ist als nur einmal zu verwendendes Produkt sehr teuer. Weiterhin ist das zugehörige Meßgerät infolge der Verwendung eines Reflektionsphotometers zur Messung der Farbstoffkonzentration relativ aufwendig.

Die faseroptische Farbstoffmessung kann darüber hinaus durch Probleme bei der optischen Messung, beispielsweise durch Auflagerungen auf dem optischen Auge des Meßkatheters, durch Dislozierung des Meßkatheters usw. beeinträchtigt werden. Schließlich ist aufgrund der Größe des Faseroptik-Thermistor-Katheters die Thermo-Dye-Dilutionsmessung nur in der Arteria femoralis möglich. Die bisher übliche Größe der Meßkatheter schließt weiterhin eine Messung bei Kindern und Säuglingen völlig aus.

Die der Erfindung zugrundeliegende Aufgabe besteht daher darin, eine Vorrichtung der eingangs genannten Art anzugeben, mit der der Füllungszustand eines Blutkreislaufs ohne Farbstoffdilution nur mittels einer Thermodilution bestimmt werden kann.

Diese Aufgabe wird gemäß der Erfindung durch die Ausbildung gelöst, die in den Patentansprüchen 1 und 2 jeweils angegeben ist.

Besonders bevorzugte Ausgestaltungen und Weiterbildungen der erfindungsgemäßen Vorrichtung sind Gegenstand der Patentansprüche 3 bis 10.

Die erfindungsgemäße Vorrichtung beruht somit darauf, daß aus der Thermodilutionskurve ein Parameter abgeleitet wird, der die Summe kleinerer Mischvolumina ohne das größte Mischvolumen repräsentiert. Diese Volumina entsprechen im wesentlichen den enddiastolischen Herzvolumina. Der entsprechende Parameter wird deshalb vereinfachend globales enddiastolisches Herzvolumen (GEDV) genannt. Das GEDV wird z.B. aus der Differenz von Gesamtmischvolumen ITTV und dem größten Mischvolumen PTV oder als eine Funktion der Erscheinungszeit AT erhalten. In Verbindung mit anderen Ergebnissen der Auswertung der Thermodilutionskurve und speziesspezifischen Zusammenhängen können damit weitere Parameter zur Beurteilung des Kreislaufsystems gewonnen werden.

Im folgenden werden anhand der zugehörigen Zeichnung das bekannte Thermo-Dye-Verfahren sowie besonders bevorzugte Ausführungsbeispiele der erfindungsgemäßen Vorrichtung näher beschrieben. Es zeigen

Fig. 1 eine schematische Darstellung einer herkömmlichen Meßanordnung zur Durchführung des Thermo-Dye-Verfahrens,
Fig. 2 eine schematische Darstellung der verschiedenen zu ermittelnden Werte nach dem Stand der Technik,
Fig. 3 den charakteristischen Verlauf der zur Ermittlung der gewünschten Volumenwerte herangezogenen Dilutionskurven, insbesondere der Thermodilutionskurven, und

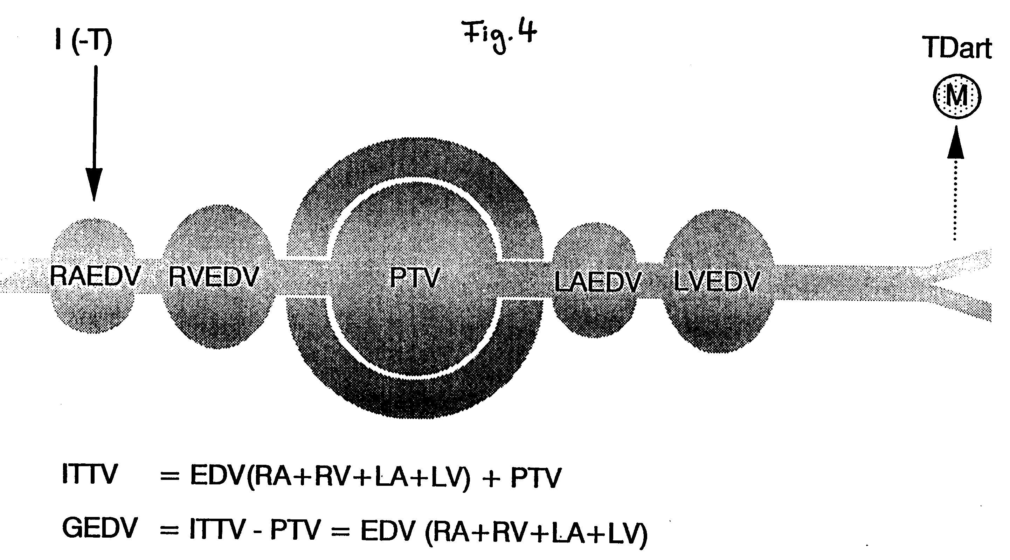

Fig. 4 eine schematische Darstellung der verschiedenen mit der erfindungsgemäßen Vorrichtung zu ermittelnden Werte.

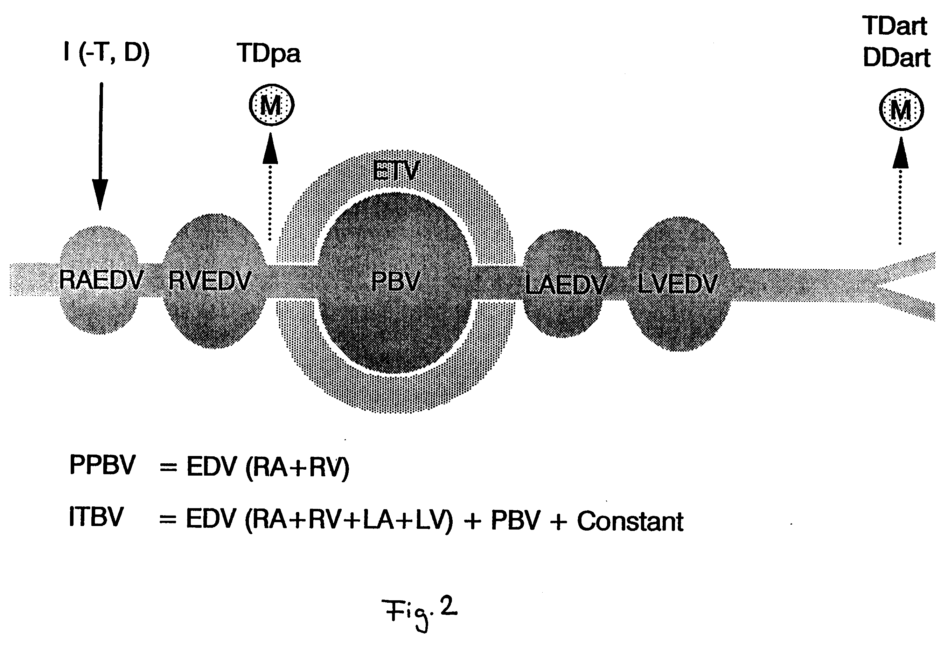

In der Praxis wird beim Thermo-Dye-Verfahren eine gekühlte Indocyaningrün-Lösung in das zentralvenöse Gefäßsystem eines Patienten injiziert. Da der Farbstoff Indocyaningrün sich sofort mit Plasmaproteinen verbindet, bleibt er zumindest während der ersten Herz-Lungen-Passage im Blutgefäßsystem (intravasaler Marker). Die eingebrachte Kälte wird zuerst analog dem Farbstoff von der rechten Herzkammer in die Lunge transportiert. Dort kann die Kälte mittels Wärmediffusion jedoch auch in das die Lungenkapillaren umgebende Gewebe eindringen. Die Kälte verteilt sich somit im intra- und im extravasalen Raum in der Lunge (sog. extravasaler Marker). Von der Lunge fließt die Kälte infolge des größeren Verteilungsvolumens in der Lunge im Vergleich zum Farbstoff verzögert zur linken Herzkammer ab. Farbstoff- und Thermodilutionskurven werden mittels eines Faseroptik-Thermistor-Katheters im arteriellen Gefäßsystem in der Arteria femoralis erfaßt (hierzu s. Fig. 2).

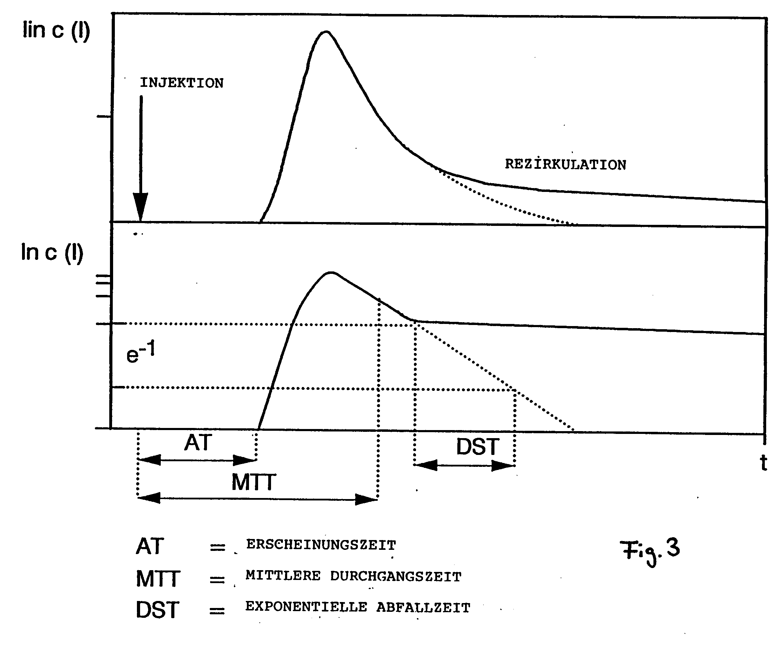

Aus der arteriellen Thermodilutionskurve TD werden das Herzzeitvolumen $C.O._{TDart}$, die Erscheinungszeit AT, die mittlere Durchgangszeit $MTD_{TDart}$ und die exponentielle Abfallzeit $DST_{TDart}$ berechnet. Aus der arteriellen Farbstoffdilutionskurve DD werden nur die Zeitparameter $MTT_{DDart}$ und $DST_{DDart}$ bestimmt. Diese Kurven sind in Fig. 3 der zugehörigen Zeichnung dargestellt.

Für die Berechnung des Herzzeitvolumens C.O. wird die Standard-Stewart-Hamiltonformel in ihrer Adaption für die Thermodilution angewandt.

Das kardiopulmonale System des Menschen stellt bezüglich der Indikatorverdünnung eine Reihenschaltung von einzelnen Mischvolumina dar, wie es in Fig. 2 schematisch gezeigt ist. Das von einem Indikator durchlaufende Gesamtvolumen berechnet sich aus dem Produkt des Herzzeitvolumens C.O. und der mittleren Durchgangszeit MTT. Das Volumen der größten Mischkammer ist durch das Produkt aus C.O. und DST gegeben.

Aus der arteriellen Thermodilutionskurve können das intrathorakale Thermovolumen ITTV und das pulmonale Thermovolumen PTV ermittelt werden, da die Lunge für die Kälte des Injektats das größte Mischvolumen darstellt:

$$ITTV = C.O._{TDart} \times MTT_{TDart}$$

$$PTV = C.O._{TDart} \times DST_{TDart}.$$

Aus der arteriellen Farbstoffdilutionskurve können das intrathorakale Blutvolumen ITBV und das pulmonale Blutvolumen PBV berechnet werden, da für den Farbstoff das größte Mischvolumen im pulmonalen Gefäßsystem liegt:

$$ITBV = C.O._{TDart} \times MTT_{DDart}$$

$$PBV = C.O._{TDart} \times DST_{DDart}.$$

Das extravasale Lungenwasservolumen EVLW kann aufgrund der Befunde aus der Literatur gleichgesetzt werden mit dem extravasalen Kälteausbreitungsvolumen in der Lunge ETV. EVLW kann über ETV auf zwei Wegen errechnet werden:

$$EVLW = ETV_{MTT} = ITTV - ITBV$$

$$und \quad EVLW = ETV_{DST} = PTV - PBV.$$

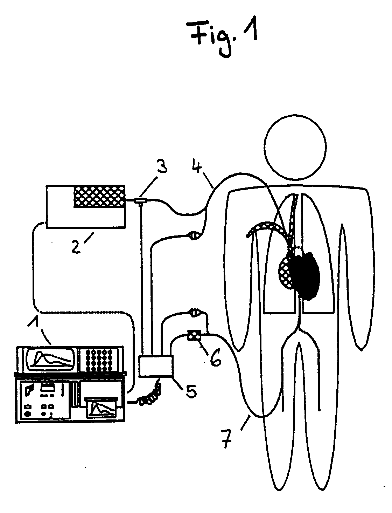

Eine herkömmliche Meßanordnung für das Thermo-Dye-Verfahren ist in Fig. 1 dargestellt. Eine derartige Meßanordnung wird beispielsweise unter der Bezeichnung COLD Z-021 hergestellt von Siemens AG, Erlangen, vertrieben. Bezüglich weiterer Einzelheiten wird auf das zugehörige Datenblatt verwiesen. Wie es in Fig. 1 dargestellt ist, umfaßt die gesamte Meßanordnung neben dem genannten COLD Z-021-System 1 einen Thermodilutionsinjektor 2, einen Temperatursensor 3, einen pulmonalarteriellen Katheter 4, einen Verteiler 5, ein optisches Modul 6 und einen femoralarteriellen faseroptischen Thermistorkatheter 7.

Üblicherweise werden im Patienten ein Pulmonaliskatheter und ein femoralarterieller Faseroptik-Thermistor-Katheter plaziert. Die gekühlte Farbstofflösung wird entweder von Hand oder mittels einer Injektionsmaschine injiziert, nachdem durch Drücken einer Taste eine Messung am Meßgerät gestartet worden ist. Während der Injektion wird mittels des Injektattemperatursensors die Temperatur des Injektates gemessen. Das Meßgerät registriert mittels des angeschlossenen, im Patienten arteriell plazierten Faseroptik-Thermistor-Katheters die Farbstoff- und Thermodilutionskurven. Anhand der oben beschriebenen Gleichungen werden die Werte für C.O., ITTV, PTV, ITBV, PBV, $ETV_{MTT}$ und $ETV_{DST}$ ermittelt. Die Ermittlung des Wertes $ETV_{DST}$

dient zur Fehlererfassung. Ein Meßfehler wird ausgegeben, wenn $ETV_{MTT}$ und $ETV_{DST}$ nicht innerhalb vorgegebener Grenzen übereinstimmen.

Bei der erfindungsgemäßen Vorrichtung werden im Gegensatz zum Thermo-Dye-Verfahren die gewünschten Werte nur mittels einer Thermodilution ohne Farbstoffdilutionsmessung ermittelt.

Dazu kann vor der Injektion der kalten Flüssigkeit der zeitliche Verlauf der Basisbluttemperatur ermittelt werden. Mittels der Methode der kleinsten Fehlerquadrate wird daraus die Basislinie für die Thermodilutionskurve extrapoliert. Alternativ kann der Verlauf der Basisbluttemperatur auch gegen Ende der Messung zur Basislinienbestimmung mit verwendet werden.

Diese Basislinienbestimmung dient zur Fehlerkorrektur, z.B. zu Rauschfehlerkorrektur, und dazu, Einflüsse aus physiologisch bedingten Schwankungen wie Körperregelvorgängen, Fieber usw. sowie von Ungenauigkeiten der Infusionsanordnung auszuschließen.

Die Auswertung wird nicht mehr durch Knopfdruck am Gerät gestartet, sondern erfolgt automatisch über eine Analyse des Ausgangssignals eines Injektatflußsensors. Aus dem zeitlichen Verlauf dieses Ausgangssignals wird auf das Vorliegen einer Injektion und ihre für den Meßzweck erforderliche Gleichmäßigkeit geschlossen. Als Flußsensor kann auch der Injektattemperatursensor verwandt werden, wenn dieser so gestaltet ist, daß er sich bei ruhendem Injektat erwärmt. Das kann durch einen Temperaturangleich an die Umgebungstemperatur, beispielsweise an die Raumtemperatur oder die Körpertemperatur oder durch Aufheizen des Sensors mittels des Meßstromes erfolgen. Der Sensor hat dazu eine kleine Wärmekapazität und eine hinreichend reproduzierbare, möglichst kurze Reaktionszeit und liefert bei einer Injektion zunächst ein exponentiell abfallendes und nach Abschluß der Injektion exponentiell ansteigendes Signal.

Zur exakten Bestimmung der mittleren Durchgangszeit MTT ist eine genaue Ermittlung des Injektionszeitpunktes erforderlich.

Es wird davon ausgegangen, daß der Untersucher die Indikatorinjektion, die in der Regel aus 10 ml eiskalter Glukoselösung besteht, gleichmäßig durchführen kann. Bei Verwendung einer Injektionsmaschine ist ohnehin eine gleichmäßige Injektion gewährleistet. Die während der Injektion aufgezeichnete Injektattemperaturkurve wird auf ihre Form hin untersucht. Die Messung wird verworfen, wenn die Kurve unregelmäßig verläuft, d.h. wenn sie beispielsweise zwei oder mehr Minima aufweist. Werden die Kriterien für eine korrekte Injektion erfüllt, dann werden aus dem zeitlichen Verlauf der Injektattemperatur während der Injektion der Anfangs- und Endpunkt der Injektion sowie das Temperaturminimum ermittelt. Bei geeigneter Sensorkonstruktion besteht der Temperaturverlauf aus einem zum Anfangszeitpunkt der Injektion beginnenden exponentiellen Temperaturabfall auf das Temperaturminimum und einem zum Endzeitpunkt der Injektion beginnenden exponentiellen Temperaturanstieg. Die Zeitkonstante des Temperaturabfalls hängt dabei von der Fließgeschwindigkeit und der Sensorkonstruktion ab. Damit sind zwei Zeitpunkte gegeben, aus denen sich die Dauer der Injektion ermitteln läßt.

Da theoretisch eine praktisch nicht realisierbare Bolusinjektion vorausgesetzt wird, wird aus dem Anfangs- und Endzeitpunkt der Injektion ein korrigierter Injektionszeitpunkt, d.h. ein Anfangszeitpunkt für die Auswertung der mittleren Durchgangzeit MTT gebildet. Dieser liegt beispielsweise in erster Näherung in der Mitte zwischen dem Anfangs- und dem Endzeitpunkt der Injektion. Anschließend werden in der oben beschriebenen, an sich bekannten Weise daraus die Werte für C.O., ITTV und PTV berechnet.

Das globale enddiastolische Volumen des Herzens $GEDV_{TDart}$ wird aus der aufgenommenen arteriellen Thermodilutionskurve als neuer für den Füllungszustand des Herzens spezifischer Parameter nach der folgenden Gleichung ermittelt:

$$GEDV_{TDart} = ITTV_{TDart} - PTV_{TDart}.$$

Das globale enddiastolische Volumen des Herzens $GEDV_{AT\ TDart}$ kann auch nach der folgenden Gleichung ermittelt werden:

$$GEDV_{AT\ TDart} = a \times (AT - VOL_{TDart}) + b$$

wobei

$$AT - VOL_{TDart} = C.O._{TDart} \times AT_{TDart}$$

und

$C.O._{TDart}$ das Herzzeitvolumen,
$AT_{TDart}$ die Erscheinungszeit und
a, b speziesspezifische Kennzahlen sind.

Die Kennzahlen a, b können über einen Vergleich des mit einem anderen bekannten Verfahren, z.B. dem Thermo-Dye-Verfahren gewonnenen Wertes GEDV mit dem ermittelten Wert $AT - VOL_{TDart}$ für die jeweilige Spezies bestimmt werden.

Sie liegen zwischen 1,0 und 3,0 ml für a und zwischen +/- 20% des normalen ITBV-Wertes (für den Menschen ca. 900 ml/m$^2$ Körperoberfläche) für b.

Diese Kennzahlen liegen für den Menschen bei etwa a = 2,0 und b = -200, wobei die Meßgröße ml ist.

Daraus kann anschließend das intrathorakale Blutvolumen $ITBV_{TDart}$ nach folgender Gleichung ermittelt werden:

$$ITBV_{TDart} = a' \times GEDV_{TDart} + b',$$

wobei a' und b' ebenfalls speziesspezifische Kennzahlen sind die zwischen 1,0 und 2,0 für a' und +/- 15% des normalen ITBV-Wertes liegen. Für den Menschen sind a' = 1,22 und b' = 109 (n = 89) und für das Schwein sind a' = 1,33 und b' = 13,3 (n = 145), wobei die Maßeinheit ml ist. Diese Werte werden analog wie die Werte a, b durch Vergleich mit nach einem anderen Verfahren ermittelten Werten bestimmt.

Das bedeutet allgemein, daß es eine speziesspezifische feste Zuordnung zwischen $ITBV_{TDART}$ und $GEDV_{TDart}$ gibt. Individuelle und krankhafte Unterschiede können dabei vernachlässigt werden.

Das pulmonale Blutvolumen $PBV_{TDart}$ ergibt sich aus der arteriellen Thermodilutionskurve dann als:

$$PBV_{TDart} = ITBV_{TDart} - GEDV_{TDart}.$$

Das extravasale Lungenwasservolumen $EVLW_{TD}$ kann aus der arteriellen Thermodilutionskurve dann nach einer der beiden folgenden Gleichungen ermittelt werden:

$$EVLW_{TD} = ETV_{MTT\,TD} = ITTV_{TD} - ITBV_{TD}, \text{ und}$$

$$EVLW_{TD} = ETV_{DST\,TD} = PTV_{TD} - PBV_{TD}.$$

Als ein weiterer neuerer Parameter wird der globale Herzfunktionsindex CFI schließlich wie folgt ermittelt:

$$CFI_{TDart} = C.O._{TDart}/GEDV_{TDart}.$$

Bei der erfindungsmäßen Vorrichtung kann ein einziger arterieller Thermodilutionskatheter verwandt werden, der an der distalen Spitze über einen kleinen elektrisch isolierten Thermistor verfügt und außerdem ein Lumen zur Blutentnahme und Messung des arteriellen Blutdruckes aufweisen kann. Dieser Katheter kann so klein ausgebildet sein, daß er problemlos in der Arteria radialis plaziert werden kann.

Die oben beschriebene Meßvorrichtung ist aufgrund der wesentlich einfacheren Gerätetechnik im Vergleich zu dem bekannten Thermo-Dye-Verfahren kostengünstiger. Sie kommt ohne aufwendige Technik und vor allem ohne Pulmonaliskatheter aus. Aufgrund der einfach und mit geringer Größe ausbildbaren Meßkathetern kann die Vorrichtung überall dort eingesetzt werden, wo ein zentralvenöser Zugang zur Indikatorinjektion liegt und gleichzeitig ein arterieller Zugang zur Temperaturmessung möglich ist.

In der Regel kann die Injektion über einen zentralvenösen Katheter erfolgen, die Plazierung der Temperatursonde über einen Arterioradialiskatheter.

Das angeschlossene Meß- und Auswertegerät ist so ausgebildet, daß es die zur Bestimmung der gewünschten Werte nach den obigen Gleichungen erforderlichen Recheneinheiten aufweist bzw. in Form eines Mikroprozessors entsprechend ausgebildet und/oder programmiert ist.

**Patentansprüche**

1. Vorrichtung zum Bestimmen des Füllungszustandes eines Blutkreislaufs mittels Thermodilution mit einer Thermodilutionsmeßanordnung, die einen Thermodilutionsinjektor, einen Temperatursensor und ein Steuer- und Auswertegerät umfaßt, an dem die Ausgangswerte des Temperatursensors und weitere Meßparameter liegen und das aus der Thermodilutionskurve das intrathorakale Thermovolumen $ITTV_{TDart}$ und das pulmonale Thermovolumen $PTV_{TDart}$ nach den Gleichungen bildet:

$$ITTV_{TDart} = C.O._{TDart} \times MTT_{TDart} \text{ und}$$

$$PTV_{TDart} = C.O._{TDart} \times DST_{TDart},$$

wobei $C.O._{TDart}$ das Herzzeitvolumen ist, das nach der Standard-Stuart-Hamilton-Formel in ihrer Adaption für die Thermodilution bestimmt wird, $MTT_{TDart}$ die mittlere Durchgangszeit ist und $DST_{TDart}$ die exponentielle Abfallzeit bezeichnet, dadurch gekennzeichnet, daß das Steuer- und Auswertegerät eine Differenzeinheit aufweist, an der die gebildeten Werte $ITTV_{TDart}$ und $PTV_{TDart}$ liegen und deren Ausgangswert $ITTV_{TDart} - PTV_{TDart}$ das globale enddiastolische Herzvolumen $GEDV_{TDart}$ ist, das die Summe der Herzkammervolumina ohne das Lungenvolumen wiedergibt.

2. Vorrichtung zum Bestimmen des Füllungszustandes eines Blutkreislaufs mittels Thermodilution mit einer Thermodilutionsmeßanordnung, die einen Thermodilutionsinjektor, einen Temperatursensor und ein Steuer- und Auswertegerät mit einer Recheneinheit umfaßt, an dem die Ausgangswerte des Temperatursensors und weitere Meßparameter liegen und das aus der Thermodilutionskurve die Erscheinungszeit $AT_{TDart}$ und das Herzzeitvolumen $C.O._{TDart}$ bildet, dadurch gekennzeichnet, daß in der Recheneinheit das Herzzeitvolumen $C.O._{TDart}$ mit der Erscheinungszeit $AT_{TDart}$ multipliziert, das Ergebnis mit einer Kennzahl a multipliziert, eine Kennzahl b hinzuaddiert und dieses Ergebnis als globales enddiastolisches Herzvolumen $GEDV_{AT\,TDart}$ ausgegeben wird, das die Summe der Herzvolumina ohne das Lungenvolumen wiedergibt, wobei im Auswertegerät Listen der speziesspezifischen Kennzahlen a und b gespeichert sind, auf die die Recheneinheit bei der Auswertung zugreift.

3. Vorrichtung nach Anspruch 1 oder 2, dadurch gekennzeichnet, daß im Auswertegerät Listen von speziesspezifischen Kennzahlen a' und b' gespeichert sind und eine Recheneinheit vorgesehen ist, die einen zugegriffenen Wert a' und den Wert $GEDV_{TDart}$ multipliziert und einen zugegriffenen Wert b' zur Bildung des intrathorakalen Blutvolumens ITBV$_{TDart}$ zuaddiert.

4. Vorrichtung nach Anspruch 3, dadurch gekennzeichnet, daß das Auswertegerät eine Subtraktionseinheit enthält, an der die Werte ITBV$_{TDart}$ und GEDV$_{TDart}$ liegen und deren Ausgangsgangswert das pulmonale Blutvolumen PBV$_{TDart}$ ist.

5. Vorrichtung nach Anspruch 3, dadurch gekennzeichnet, daß das Auswertegerät eine Subtraktionseinheit enthält, an der die Werte ITTV$_{TDart}$ und ITBV$_{TDart}$ als Eingangswerte liegen und deren Ausgangswert das extravasale Lungenwasservolumen EVLW$_{TDart}$ wiedergibt.

6. Vorrichtung nach Anspruch 4, dadurch gekennzeichnet, daß das Auswertegerät eine Subtraktionseinheit enthält, an der die Werte PTV$_{TDart}$ und PBV$_{TDart}$ als Eingangswerte liegen und deren Ausgangswert das extravasale Lungenwasservolumen EVLW$_{TDart}$ wiedergibt.

7. Vorrichtung nach Anspruch 1, dadurch gekennzeichnet, daß das Auswertegerät eine Divisionseinheit enthält, an der das Herzzeitvolumen C.O$_{TDart}$ und der Wert GEDV$_{TDart}$ als Eingangswerte liegen, und deren Ausgangswert den globalen Herzfunktionsindex wiedergibt.

8. Vorrichtung nach einem der vorhergehenden Ansprüche, gekennzeichnet durch einen Injektatflußsensor, wobei das Auslösen einer Messung über eine Analyse des zeitlichen Verlaufs seines Ausgangssignals gesteuert wird.

9. Vorrichtung nach Anspruch 8, dadurch gekennzeichnet, daß ein Injektattemperatursensor zur Verwendung auch als Flußsensor so gestaltet ist, daß er sich bei ruhendem Injektat erwärmt.

10. Vorrichtung nach Anspruch 9, dadurch gekennzeichnet, daß der Injektattemperatursensor durch Aufheizung mittels eines Meßstromes erwärmt wird.

**Claims**

1. Device for determining the fill level of blood circulation by means of thermodilution, having a thermodilution measuring arrangement which comprises a thermodilution injector, a temperature sensor and a control and evaluation apparatus, at which the output values of the temperature sensor and further measuring parameters lie and which from the thermodilution curve forms the intrathoracic thermovolume ITTV$_{TDART}$ and the pulmonary thermovolume PTV$_{TDart}$ according to the following equations:

$$ITTV_{TDART} = C.O._{TDart} \times MTT_{TDart} \text{ and}$$

$$PTV_{TDart} = C.O._{TDart} \times DST_{TDart},$$

in which C.O.$_{TDart}$ is the cardiac output, which is determined according to the Standard-Stuart-Hamilton formula in its adaptation to thermodilution. MTT$_{TDart}$ is the mean transit time and DST$_{TDart}$ denotes the exponential fall time, characterised in that the control and evaluation apparatus comprises a differential unit, at which the values ITTV$_{TDart}$ and PTV$_{TDart}$ that have been formed lie and the output value ITTV$_{TDart}$ - PTV$_{TDart}$ of which is the global end diastolic volume of the heart GEDV$_{TDart}$, which represents the sum of the cardiac chamber volumes without the volume of the lungs.

2. Device for determining the fill level of blood circulation by means of thermodilution, having a thermodilution measuring arrangement which comprises a thermodilution injector, a temperature sensor and a control and evaluation apparatus with a processor, at which control and evaluation apparatus the output values of the temperature sensor and further measuring parameters lie and which from the thermodilution curve forms the appearance time AT$_{TDart}$ and the cardiac output C.O.$_{TDart}$, characterised in that in the processor the cardiac output C.O.$_{TDart}$ is multiplied by the appearance time AT$_{TDart}$, the result is multiplied by a characteristic value $\underline{a}$, a characteristic value $\underline{b}$ is added and that result is read out as global end diastolic volume of the heart GEDV$_{ATTDart}$, which represents the sum of the volumes of the heart without the volume of the lungs, wherein in the evaluation apparatus there are stored lists of species-specific characteristic values $\underline{a}$ and $\underline{b}$, which the processor accesses during the evaluation.

3. Device according to claim 1 or 2, characterised in that lists of species-specific characteristic values a' and b' are stored in the evaluation apparatus and a processor is provided which multiplies an accessed value a' and the value GEDV$_{TDart}$ and adds an accessed value b' to form the intrathoracic blood volume ITBV$_{TDart}$.

4. Device according to claim 3, characterised in that the evaluation apparatus contains a subtraction unit, at which the values ITBV$_{TDart}$ and GEDV$_{TDart}$ lie and the output value of which is the pulmonary blood volume PBVT$_{TDart}$.

5. Device according to claim 3, characterised in that the evaluation apparatus contains a subtraction unit, at which the values ITTV$_{TDart}$ and ITBV$_{TDart}$ lie as input values and the output value of which represents the extravascular volume of lung fluid EVLW$_{T}$-

Dart.

6. Device according to claim 4, characterised in that the evaluation apparatus contains a subtraction unit, at which the values $PTV_{TDart}$ and $PBV_{TDart}$ lie as input values and the output value of which represents the extravascular volume of lung fluid $ELVW_{TDart}$.

7. Device according to claim 1, characterised in that the evaluation apparatus contains a division unit, at which the cardiac output $C.O._{TDart}$ and the value $GEDV_{TDart}$ lie as input values, and the output value of which represents the global cardiac function index.

8. Device according to any one of the preceding claims, characterised by an injected substance flow sensor, the initiation of a measurement being controlled by way of an analysis of the time characteristic of its output signal.

9. Device according to claim 8, characterised in that an injected substance temperature sensor for use also as flow sensor is designed so that it becomes warm when the injected substance is at rest.

10. Device according to claim 9, characterised in that the injected substance temperature sensor is warmed by being heated by means of a measuring current.

**Revendications**

1. Dispositif pour déterminer le niveau de remplissage d'un système circulatoire sanguin, par thermodilution à l'aide d'un dispositif de mesure de la thermodilution qui comprend un injecteur de thermodilution, un capteur de température et un appareil de commande et d'analyse indiquant les valeurs de sortie du capteur de température et d'autres paramètres de mesure, lequel appareil de commande et d'analyse forme, à partir de la courbe de thermodilution, le thermovolume intrathoracique $ITTV_{TDart}$ et le thermovolume pulmonaire $PTV_{TDart}$ d'après les équations :

$$ITTV_{TDart} = C.O._{TDart} \times MTT_{TDart} \text{ et}$$

$$PTV_{TDart} = C.O._{TDart} \times DST_{TDart},$$

où $C.O._{TDart}$ est le volume de temps cardiaque qui est déterminé d'après la formule standard de *Stuart* et *Hamilton* dans son adaptation pour la thermodilution, $MTT_{TDart}$ étant le temps de passage moyen et $DST_{TDart}$ désignant le temps de diminution exponentiel, caractérisé

en ce que l'appareil de commande et d'analyse présente une unité différentielle sur laquelle se trouvent les valeurs formées $ITTV_{TDart}$ et $PTV_{TDart}$ et dont la valeur de sortie $ITTV_{TDart}\text{-}PTV_{TDart}$ est le volume cardiaque global $GEDV_{TDart}$ en fin de diastole, lequel volume cardiaque reproduit la somme des volumes des ventricules du coeur sans le volume pulmonaire.

2. Dispositif pour déterminer le niveau de remplissage d'un système circulatoire sanguin, par thermodilution à l'aide d'un dispositif de mesure de la thermodilution qui comprend un injecteur de thermodilution, un capteur de température et un appareil de commande et d'analyse doté d'une unité de calcul, appareil de commande et d'analyse indiquant les valeurs de sortie du capteur de température et d'autres paramètres de mesure et qui forme, à partir de la courbe de thermodilution, la durée du phénomène $AT_{TDart}$ et le volume de temps cardiaque $C.O._{TDart}$,
caractérisé
en ce que, dans l'unité de calcul, le volume de temps cardiaque $C.O._{TDart}$ est multiplié par la durée du phénomène $AT_{TDart}$, en ce que le résultat est multiplié par un nombre caractéristique $a$, en ce qu'un nombre caractéristique $b$ est ajouté et en ce que ce résultat est donné en tant que volume cardiaque $GEDV_{ATTDart}$ global en fin de diastole, lequel volume cardiaque reproduit la somme des volumes cardiaques sans le volume pulmonaire, où des listes des nombres caractéristiques spécifiques $a$ et $b$ sont stockées dans l'appareil d'analyse, listes auxquelles l'unité de calcul a accès lors de l'analyse.

3. Dispositif selon la revendication 1 ou 2, caractérisé en ce que des listes de nombres caractéristiques spécifiqrnes $a'$ et $b'$ sont stockées dans l'appareil d'analyse, et en ce qu'il est prévu une unité de calcul qui multiplie une valeur $a'$, à laquelle l'unité de calcul a accès, et la valeur $GEDV_{TDart}$, et en ce que ladite unité de calcul ajoute une valeur $b'$ à laquelle elle a accès pour former le volume sanguin intrathoracique $ITBV_{TDart}$.

4. Dispositif selon la revendication 3, caractérisé en ce que l'appareil d'analyse contient une unité de soustraction indiquant les valeurs $ITBV_{TDart}$ et $GEDV_{TDart}$ et dont la valeur de sortie est le volume sanguin pulmonaire $PBV_{TDart}$.

5. Dispositif selon la revendication 3, caractérisé en ce que l'appareil d'analyse contient une unité de soustraction indiquant les valeurs $ITTV_{TDart}$ et $ITBV_{TDart}$ servant de valeurs d'entrée, et dont la valeur de sortie reproduit le volume d'eau pulmonaire $EVLW_{TDart}$, hors des vaisseaux sanguins.

6. Dispositif selon la revendication 4, caractérisé en ce que l'appareil d'analyse contient une unité de soustraction indiquant les valeurs $PTV_{TDart}$ et $PBV_{TDart}$ servant de valeurs d'entrée, et dont la valeur de sortie reproduit le volume d'eau pulmonaire $EVLW_{TDart}$, hors des vaisseaux sanguins.

7. Dispositif selon la revendication 1, caractérisé en ce que l'appareil d'analyse contient une unité de division indiquant le volume de temps cardiaque $C.O._{TDart}$ et la valeur $GEDV_{TDart}$ servant de valeurs d'entrée, et dont la valeur de sortie reproduit l'indice global de la fonction cardiaque.

8. Dispositif selon l'une quelconque des revendications précédentes, caractérisé par un capteur de débit d'injection, où le déclenchement d'une mesure est commandé par une analyse de l'évolution dans le temps de son signal de sortie.

9. Dispositif selon la revendication 8, caractérisé en ce qu'un capteur de température d'injection, pour être utilisé, est configuré également comme un capteur de débit, de façon telle qu'il se réchauffe lorsque l'injection est au repos.

10. Dispositif selon la revendication 9, caractérisé en ce que le capteur de température d'injection est réchauffé en chauffant au moyen d'un courant de mesure.

Fig. 1

$$PPBV = EDV (RA+RV)$$

$$ITBV = EDV (RA+RV+LA+LV) + PBV + Constant$$

Fig. 2

lin c (l)

INJEKTION

REZİRKULATION

ln c (l)

e⁻¹

AT

DST

MTT

AT    =  ERSCHEINUNGSZEIT

MTT   =  MITTLERE DURCHGANGSZEIT

DST   =  EXPONENTIELLE ABFALLZEIT

t

Fig. 3

Fig. 4

$$ITV = EDV(RA+RV+LA+LV) + PTV$$

$$GEDV = ITTV - PTV = EDV(RA+RV+LA+LV)$$